# EUROPEAN PATENT APPLICATION

(11) **EP 3 614 143 A1**
(43) Date of publication of application: **26.02.2020**
(21) Application number: 18190073.9
(22) Date of filing: 21.08.2018
(51) Int. Cl.: G01N 33/18

(54) **METHOD AND DEVICE TO DETERMINE THE HARDNESS OF WATER AND IEX MATERIAL USED IN THIS METHOD**

(71) Applicant: BWT AKTIENGESELLSCHAFT, 5310 Mondsee (AT)
(72) Inventor: BALIDAS, Pierre, Bloomfield, NJ 07003 (US); BRAND, Christian, 68350 Brunstatt (FR); JOHANN, Jürgen, 69226 Nußloch (DE)
(74) Representative: Patentanwaltskanzlei Cartagena

(57) **Abstract**

In a method to determine hardness of water, a sample of the water is taken, the sample is treated by adding an ion exchange (IEX) material to the sample which is configured to exchange at least one hardness causing ion species in the sample by another ion species, resulting in a change of an electrical property of the sample, the change of the electrical property is quantified by conducting a measurement of the electrical property in the treated sample and a reference measurement of the electrical property in an untreated reference sample and the quantified change of the electrical property is used as an indicator of the hardness of the water. An IEX material configured to be used in this method has a moisture content of 1 to 25 wt% and is contained in a moisture resistant, closed container in an amount of lg to 50g. A device to conduct the method is configured to receive result data from the measurements and comprises a data processing unit which is configured to process the result data to quantify a change of the electrical property and a display unit which is coupled to the data processing unit and is configured to display the water hardness.

## Description

The present invention refers to a method and a device to determine the hardness of water and to an IEX (ion exchange) material.

Total hardness of water is the sum of the concentrations of cations of alkaline earth metals in water. In practice, the total hardness mostly corresponds to the sum of the concentrations of calcium and magnesium ions in water. The concentrations of other earth metal ions in water is usually negligible.

Many households are supplied with tap water with a high content of calcium and magnesium. Knowing the hardness of tap water is important because it affects the performance and service life of electrical appliances such as washing machines, hot water appliances or dishwashers.

A common procedure for measuring water hardness is titration. The amount of titration solution required to bind the calcium and magnesium ions is measured. Suitable titration solutions are freely available on the market. The titration solution is dropped into a water sample until its color changes. The amount of titration solution required is proportional to the hardness of the water.

Even easier is the use of colorimetric test kits. Usually such test kits contain test strips. The addition of water to these test strips causes a color reaction. The hardness of water can be determined by comparing the discolored test strips with a comparison color scale.

Those types of hardness measurements are very easy to conduct but have a few significant inconveniences. The lifetime of such titration solutions and test kits is limited. After expiration their use will deliver unprecise results, even if they were stored properly and kept closed. Once opened the lifetime is also limited, especially depending on the storage conditions. In some cases the color change can finally be difficult to detect properly, leading to poor results accuracy.

The present invention is based on the object to develop an improved method to determine water hardness.

This object is achieved by the method of claim 1, the IEX material of claim 7 and the device to determine water hardness having the features of claim 8. Preferred embodiments of the method are specified in dependent claims 2 to 9. A preferred embodiment of the device is specified in dependent claim 9.

The present invention is based on the understanding that the results of measurements of an electrical property of the water before and after being exposed to an IEX material may serve as an indicator for the total hardness of the water.

The method according to the invention comprises the steps of
a. Taking a sample of the water,
b. Treating the sample by adding an ion exchange (IEX) material to the sample which is configured to exchange at least one hardness causing ion species in the sample by another ion species, resulting in a change of an electrical property of the sample,
c. Quantifying the change of the electrical property by conducting a measurement of the electrical property in the treated sample and a reference measurement of the electrical property in an untreated reference sample and
d. Using the quantified change of the electrical property as an indicator of the hardness of the water.

The method according to the invention is suitable for all types of hardness containing water including water of a typical hardness in the range from 5 °fH to 65 °fH (°fH = French degrees).

In contrast to the prior art, the method according to the invention is based on a physical measurement of a property change which results from a chemical reaction, namely said ion exchange. Compared to the above mentioned prior art methods, the method according to the invention delivers accurate results which are independent from the lifetime of titration solutions and test kits. The only reagent used is the IEX material which can be kept functional even under challenging storage conditions, as will be explained below.

To ensure good reproducibility and accuracy of the measurements, it is preferred that the measurements are always conducted with the same amount of water. Wherein the absolute amount of the samples is basically irrelevant, it is preferred that for each determination of a water hardness the treated sample and the untreated sample contain an identical volume of the water. Further, preferably also measurements of separate determinations are always conducted with identical sample volumes.

For the same reason it is preferred to add always the same amount of IEX material to the samples.

Generally, it is preferred to conduct steps a. to c. above always under the same conditions. Parameters like ambient temperature, temperature of the probe, amount and properties of the IEX material etc. shall be kept constant during the measurements. Ideally, measurements, also measurements within separate determinations, are always conducted under the same or at least under comparable conditions.

If it is not possible to keep parameters constant, compensatory measures may be preferable. For example, a temperature compensation may be advisable to compensate for deviations from room tem-perature.

Sample volumes can be set for example to a value in the range from 1 to 1000 ml. It is preferred that the size of the samples is in the range from 1 to 500 ml, more preferably in the range from 1 to 250 ml.

In preferred embodiments the sample and the untreated sample are identical. This means, that the reference measurement of the electrical property is conducted in the untreated reference sample, followed by the addition of the IEX material to this sample and further followed by the measurement of the electrical property in this (treated) sample. Alternatively, the reference measurement can be carried out in a separate, untreated sample.

In preferred embodiments the method is characterized by at least one of the following additional fea-tures:
a. The IEX material is configured to exchange calcium and/or magnesium ions against sodium and/or potassium ions.
b. The IEX material is a strong acid cationic (SAC) IEX material.

A particularly preferred IEX strong acid cationic (SAC) IEX material is regenerated under the potassium form. During the treatment, calcium and magnesium ions are exchanged against potassium ions. The exchange reaction takes place because the IEX material exhibits a higher affinity to calcium and magnesium ions than to potassium ions. The following reaction occurs during the treatment of the sample:

2 R-SO₃-K + M²⁺ → (R-SO₃)₂-M + 2 K⁺

In this equation, M can be Ca or Mg.

It is preferred that the IEX resin material is an IEX resin.

In preferred embodiments the method is characterized by at least one of the following additional fea-tures:
a. The IEX material has a moisture content in the range from 1 to 25 wt%.
b. The IEX material has a moisture content in the range of 2 to 20 wt%.
c. The IEX material has a moisture content in the range of 5 to 10 wt%.

Feature c is a particularly preferred feature.

The low moisture content has a positive influence on the storage stability of the IEX material, which is described in more detail below.

Preferably the amount of IEX material added should provide at least a stoichiometric ratio of the total amount of the hardness causing ions in the sample to the capacity of the added IEX material to adsorb these ions, otherwise not all hardness causing ions can be bound to the IEX material and thus the electrical properties of the treated sample cannot be determined correctly.

In particularly preferred embodiments the method is characterized by at least one of the following additional features:
a. The IEX material is added in an amount to provide a ratio of the total amount of the hardness causing ions in the sample to the capacity of the added IEX material to adsorb these ions in the range from 1 to 0,002. In other words, preferably the capacity of the added IEX material exceeds the total amount of hardness in the sample by a factor of up to 500.
b. The IEX material is added in an amount to provide a ratio of the total amount of the hardness causing ions in the sample to the capacity of the added IEX material to adsorb these ions in the range from 0,5 to 0,004. In other words, preferably the capacity of the added IEX material exceeds the total amount of hardness in the sample by a factor in the range from 2 to 250.
c. The IEX material is added in an amount to provide a ratio of the total amount of the hardness causing ions in the sample to the capacity of the added IEX material to adsorb these ions in the range from 0,1 to 0,01. In other words, preferably the capacity of the added IEX material exceeds the total amount of hardness in the sample by a factor in the range from 10 to 100.

Feature c is a particularly preferred feature.

In further preferred embodiments the method is characterized by at least one of the following additional features:
a. the electrical property is an electrical conductivity.
b. the electrical property is an electrical resistance.

Feature a. is particularly preferred.

It is preferred that the measured electrical property of the treated sample and that of the untreated sample is in both cases the electrical conductivity.

In further preferred embodiments the method is characterized by at least one of the following additional steps:
a. In order to quantify the change of the electrical property, a difference of the electrical property in the treated sample and the electrical property in the untreated reference sample is determined.
b. The total hardness of the water is determined as a linear function of the difference.
c. The total hardness of the water is determined from a correlation table or correlation chart.

A preferred approach to quantify the change of the electrical property is to determine the difference between the results of the two measurements and to correlate this difference with a value for total hardness.

As will be explained below, the ratio between the water hardness and the difference is linear. Consequently the difference is a direct indicator of water hardness, which can be determined by a calculation (according to feature b.) or by comparison with reference values in said correlation table or correlation chart.

It is preferred that the temperature of the samples is constant during the measurements. If it is not possible to keep the temperature constant or if there is simply a deviation from a predefined measurement temperature, for example a deviation from room temperature, it may be preferable that the measurement of the electrical property takes place with compensation of the temperature. State-of-the-art temperature-compensated electrodes are known for this purpose.

A part of the present invention is an IEX material suitable for use in the described method. This IEX material comprises the following additional features:
a. the IEX material has a moisture content of 1 to 25 wt% and
b. the IEX material is contained in a moisture resistant, closed container and
c. the IEX material is contained in the container in an amount of 1 g to 50 g.

Usually a container contains exactly the amount of IEX material required to treat one sample.

Preferred embodiments of the IEX material have already been described above and are not mentioned here again in order to avoid repetition. In order to provide moisture resistance the container preferably comprises a film, more preferably a multilayer film having a metal layer. Such metal layers usually serve as humidity barrier.

The device to determine water hardness comprises the following additional features:
a. the device is configured to receive result data from measurements of an electrical property of a sample of water,
b. the device comprises a data processing unit which is configured to process the result data to quantify a change of the electrical property and
c. the device comprises a display unit which is coupled to the data processing unit and is configured to display the water hardness.

With the device, it is possible to determine water hardness according to the method described above.

In a preferred embodiment the device is characterized by at least one of the following additional fea-tures:
a. The device comprises a pair of electrodes which are configured to measure the electrical property of the sample of water.
b. The device comprises a measuring cell for the sample.
c. The measuring cell comprises at least one marking for a (maximum) fill level of the cell.
d. The measuring cell is configured to receive a volume of the sample of 1 - 1000 ml.

In the simplest conceivable embodiment the device is or comprises a portable electronic device, for example like a smartphone, which is configured to perform said tasks of receiving the data from the measurements, processing the result data and displaying the water hardness. The configuration of the electronic device is preferably accomplished by a suitable software configuration, for example via a mobile app which is installed on the portable electronic device.

The result data of the measurements of the electrical properties can be transmitted for example via a cable but also contactless. For this purpose the portable device may be equipped with a suitable receiving unit, for example a WLAN or Bluetooth chip. A suitable transmission unit may be coupled to the pair of electrodes.

The data processing unit can be used to calculate the difference between the results of the measurements of the electric property and to calculate the total hardness of the water as a linear function of this difference or to determine the total hardness of the water by correlation. The device may comprise a data storage unit to store the necessary a correlation data or an equation/algorithm to derive water hardness from the calculated difference.

In another embodiment it is also possible that the processing of the result data comprises the step of sending the data to an external data processing unit. This allows the result data to be stored and/or evaluated externally, for example on an internet server (cloud-computing).

### Detailed description of the invention / Working example.

Further features and advantages of the invention can be derived from the figures and the following detailed description of preferred embodiments. The preferred embodiments described are merely for the purposes of illustration and to give a better understanding of the invention and shall not in any way constitute a restriction.
Fig. 1 illustrates the general process of ion exchange in a water sample using a SAC IEX material.
Fig. 2 shows an exemplary setup to perform the total hardness measurement according to the invention, ready for a reference measurement.
Fig. 3 shows the setup of Fig. 2 ready for a measurement of the electrical conductivity of the treated sample.
Fig. 4 shows the reference curve for water hardness in connection with the difference between the treated sample and the untreated sample.
Fig. 5 illustrates the correlation between the amount of ion exchange material in a water sample and change of electrical conductivity of the sample after treatment with the ion exchange material.

### (1) General process description

The total hardness concentration of a water sample is determined by conductivity difference, before and after being exposed to a strong cationic ion exchange resin, regenerated under the potassium form. When exposed to the ion exchange resin, the calcium and magnesium ions (total hardness) from the water sample are exchanged against potassium. The exchange reactions are made possible due to the fact the IEX resin exhibits a higher affinity / selectivity for calcium and magnesium than potassium. The following reactions occur during exposition of the water to the ion exchange resin, which capacity is higher than the total hardness present in the water sample:

2 R-SO₃-K + Ca²⁺ → (R-SO₃)₂-Ca + 2 K⁺

2 R-SO₃-K + Mg²⁺ → (R-SO₃)₂-Mg + 2 K⁺

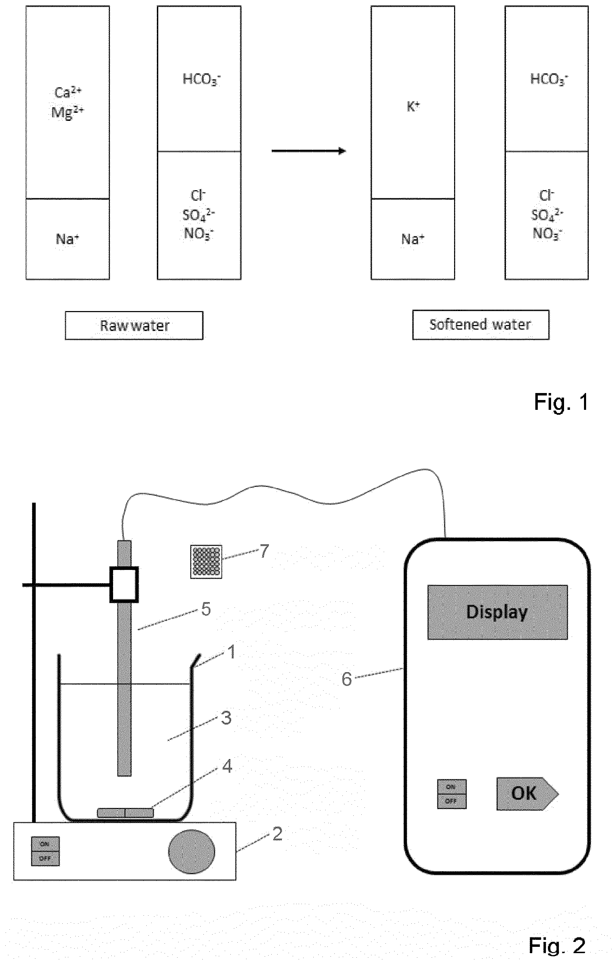

The total hardness (calcium and magnesium) present in the raw water is replaced by potassium to produce softened water. This process is illustrated in Fig. 1.

The softened water with potassium ions replacing the total hardness has a higher conductivity than the raw water sample. The potassium ion leads to a higher conductivity than the calcium and magnesium ions, due to its higher ionic molar conductivity. The amount of potassium ions released by the ion exchange resin is directly proportional to the total hardness concentration present in the raw water.

### (2) Detailed process description

The setup (see Fig. 2) comprises a beaker 1 as measuring cell, placed on a magnetic stirrer 2. In the beaker 100 ml of a water sample 3 is located, together with a spin bar 4. A temperature-compensated conductivity probe 5 is immersed in the sample 3 to measure its conductivity. It is connected to a portable electronic device 6 via a cable to transmit result data from the conductivity measurements. A packaged IEX material 7 regenerated under potassium form (ex.: 4 g) is ready for use.

The portable device 6 includes a data processing unit which is configured to process the result data. The device comprises a display unit which can show the measured conductivity. The device can comprises an "OK button" as operating element.

After pouring the water sample 3 into the beaker and once the conductivity reading from the probe 5 with temperature compensation is stable, the conductivity from the raw water is recorded by the measurement device (ex: EC1 = 654 µS/cm). To continue, the OK button is pressed.

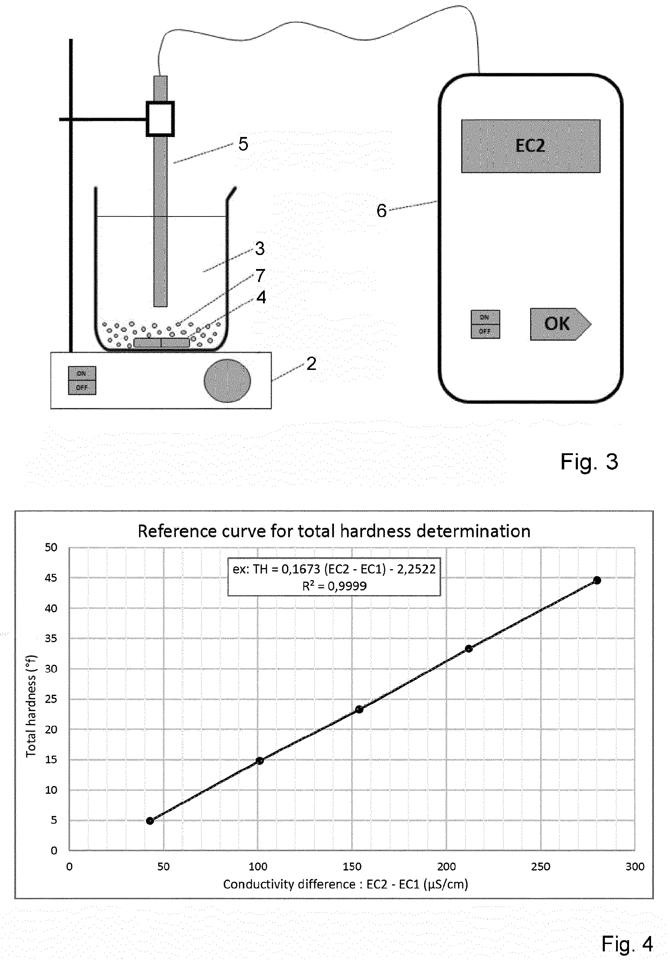

In a next step, as shown in Fig. 3, the IEX material 7 is introduced into the measurement cell. The IEX material and the sample are mixed together. Once the conductivity reading from the probe 5 is stable, the conductivity from the treated water is recorded by the device 6 (ex: EC2 = 833 µS/cm). To continue, the OK button is pressed.

From the recorded measurements, the device 6 is finally able to determine the total hardness concentration according to the curve shown in Fig. 4.

The total hardness concentration is a linear function of the conductivity difference between the treated water and the raw water (ex: 833 - 654 = 179 µS/cm for the given experimental conditions (with same ratio amount of water sample to ion exchange material). The equation is integrated in the device 6 to calculate the total hardness concentration from the conductivity difference. The parameters from the equation depends on the ratio between the water volume and the IEX material amount (ex: TH = 0,1673 x 179 - 2,2522 = 27,7 °fH). The calculated TH value is finally shown on the display of device 6.

### (3) Ratio of the IEX material to the total amount of the hardness causing ions in the sample

The amount of potassium ions released by the ion exchange material is directly proportional to the total hardness concentration present in the raw water. In order to obtain a complete exchange of the total hardness by potassium, the ion exchange material must be in excess. Its capacity must be higher than the total hardness available in the water sample for the exchange to be complete. Once the ion exchange material capacity is exceeding the total hardness amount present in the water sample, the conductivity difference continues to increase. According to the ion exchange material capacity, passed a ratio of 1 the conductivity difference should be stable.

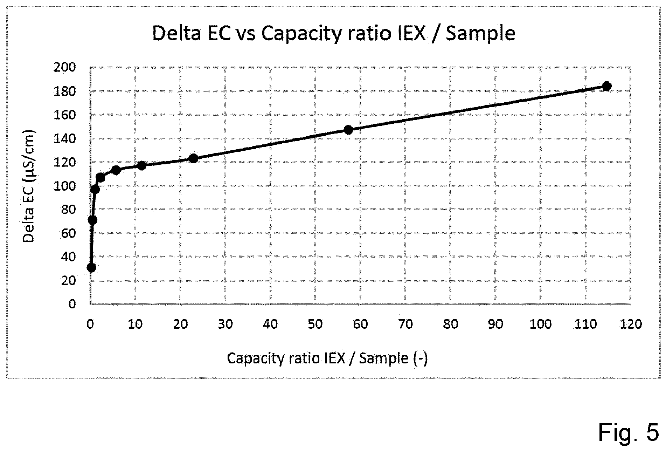

Fig. 5 shows that passed this point the conductivity difference increases proportionally to an ion exchange resin capacity to hardness ratio. In addition to the potassium ions fixed on the resin ionic groups during the regeneration (corresponding to the ion exchange resin capacity), the ion exchange resin can also sorb electrolytes such as KCl. The fact the conductivity difference is increasing passed a capacity ratio of 1 is due to the release of electrolytes such as KCl by the ion exchange resin, in order to reach an equilibrium between the electrolytes present in the resin and the solution.

In order to obtain a reliable and accurate measurement, the amount of ion exchange resin should be chosen that its capacity is in excess compared to the hardness concentration corresponding to the high end of the measurement range. In particular by always applying the same measurement conditions, sample volume to amount of ion exchange resin, it is possible obtain a conductivity difference between the raw water and the softened water which is proportional to the total hardness concentration of the raw water. Therefore by measuring the conductivity difference before and after exposition to the ion exchange resin for the same defined conditions, it is possible to determine the total hardness concentration of the raw water sample.

### (4) Ion exchange resin conditioning

To ensure a good reproducibility of the measurement, the ion exchange resin conditioning procedure is of importance. From one batch to another, the IEX resin must be conditioned identically to present similar characteristics. For each measurement, the exact same amount of watersample and ion exchange resin should be used.

The ion exchange resin can be conditioned under standard ion exchange regeneration conditions. An example procedure is presented as follow. The ion exchange resin is regenerated with a 10 % KCl solution. The amount of solution used is equivalent to 240 g of KCl per liter of ion exchange resin, at a flow rate of 3 bed volumes per hour. The ion exchange resin is then rinsed with 2 bed volumes of deionized water, at same flow rate. A final rinse is performed with 4 bed volumes of deionized water, at a flow rate of 12,5 bed volumes per hour. The exact same amount of regenerant and rinsing water per liter of ion exchange resin at similar flow rates must be used from one batch to another to ensure similar regeneration conditions and performance of the ion exchange resin.

To avoid a decline in the ion exchange resin physical properties, suppliers usually recommend to store the resin at a moisture content of about 45 to 50 % and a temperature from 2 to 40°C. It is also recommended to avoid air contact that could lead to resin drying and shrinking. Once rehydrated such resin could break due to rapid swelling of the beads. Under appropriated conditions a storage time of 5 years without affecting the performance of the resin can be expected for a strong ion exchange cationic resin.

For the purpose of the method according to the invention, the ion exchange resin is preferably dried after the regeneration, with a heater under vacuum to reach a 5 to 10 % moisture content. Cationic resin can usually withstand temperatures up to 80 °C. By drying the ion exchange resin prior to packing it in small sealed bags, it can be stored with less risk of affecting its performance and the results obtained in the total hardness determination overtime.

## Claims

1. Method to determine hardness of water, wherein
a. a sample of the water is taken and
b. the sample is treated by adding an ion exchange (IEX) material to the sample which is configured to exchange at least one hardness causing ion species in the sample by another ion species, resulting in a change of an electrical property of the sample and
c. the change of the electrical property is quantified by conducting a measurement of the electrical property in the treated sample and a reference measurement of the electrical property in an untreated reference sample and
d. the quantified change of the electrical property is used as an indicator of the hardness of the water.

2. Method according to claim 1 with at least one of the following additional steps and/or features:
a. The IEX material is configured to exchange calcium and/or magnesium ions against sodium and/or potassium ions.
b. The IEX material is a strong acid cationic (SAC) IEX material.

3. Method according one of the preceding claims with at least one of the following additional steps and/or features:
a. The IEX material has a moisture content in the range from 1 to 25 wt%.
b. The IEX material has a moisture content in the range of 2 to 20 wt%.
c. The IEX material has a moisture content in the range of 5 to 10 wt%.

4. Method according to claim 1 with at least one of the following additional steps and/or features:
a. The IEX material is added in an amount to provide a ratio of the total amount of the hardness causing ions in the sample to the capacity of the added IEX material to adsorb these ions in the range from 1 to 0,002.
b. The IEX material is added in an amount to provide a ratio of the total amount of the hardness causing ions in the sample to the capacity of the added IEX material to adsorb these ions in the range from 0,5 to 0,004.
c. The IEX material is added in an amount to provide a ratio of the total amount of the hardness causing ions in the sample to the capacity of the added IEX material to adsorb these ions in the range from 0,1 to 0,01.

5. Method according to one of the preceding claims with one of the following additional steps and/or features:
a. The electrical property is an electrical conductivity.
b. The electrical property is an electrical resistance.

6. Method according to one of the preceding claims with at least one of the following additional steps and/or features:
a. In order to quantify the change of the electrical property, a difference of the electrical property in the treated sample and the electrical property in the untreated reference sample is determined.
b. The total hardness of the water is determined as a linear function of the difference.
c. The total hardness of the water is determined from a correlation table or correlation chart.

7. An IEX material with the following features:
a. The IEX material has a moisture content of 1 to 25 wt% and
b. The IEX material is contained in a moisture resistant, closed container and
c. The IEX material is contained in the container in an amount of 1 to 50 g.

8. Device to determine water hardness, in particular according to a method according to one of the preceding claims, wherein
a. the device is configured to receive result data from measurements of an electrical property of a sample of water,
b. the device comprises a data processing unit which is configured to process the result data to quantify a change of the electrical property and
c. the device comprises a display unit which is coupled to the data processing unit and is configured to display the water hardness.

9. Device according to claim 8 with at least one of the following additional features:
a. The device comprises a pair of electrodes which are configured to measure the electrical property of the sample of water.
b. The device comprises a measuring cell for the sample.
c. The measuring cell comprises at least one marking for a (maximum) fill level of the cell.
d. The measuring cell is configured to receive a volume of the sample of 1 - 1000 ml.
